# EUROPEAN PATENT APPLICATION

(11) **EP 4 628 129 A2**
(43) Date of publication of application: **08.10.2025**
(21) Application number: 25197353.3
(22) Date of filing: 18.01.2023
(51) Int. Cl.: A61M 15/00

(54) **DRY POWDER INHALATION DEVICE**

(30) Priority: 18.01.2022 GB 202200559
(62) Divisional of application: 23706438.1
(71) Applicant: Merxin Ltd, King's Lynn, Norfolk PE30 5FQ (GB)
(72) Inventor: Purkins, Graham, King's Lynn, PE32 1EN (GB); Howgill, Stephen, Thurcaston, LE7 7JL (GB); Hodson, Peter, Breaston, DE72 3EL (GB); Stuart, Adam, King s Lynn, PE32 1BS (GB); Antoniak, Dylan, King's Lynn, PE30 3UW (GB); Barrett, Adam, King s Lynn, PE33 0PR (GB)
(74) Representative: Basck Limited

(57) **Abstract**

Disclosed is a dry powder inhalation device (300) and an insert (100) for use in a mouthpiece (307) of a dry powder inhalation device of the prior art. The disclosed dry powder inhalation device comprises a means to aerosolize powdered medicament and introduce the aerosolized medicament to an airflow directed to a user outlet (318). The dry powder inhalation device further comprises a primary deagglomeration structure (305) in the form of a baffle (305) through which the airflow is arranged to pass downstream of the means to aerosolize the powdered medicament and a secondary deagglomeration structure (301) which is a tortuous passage (310) through which the airflow is arranged to pass downstream of the primary deagglomeration structure. The deagglomeration structures are configured to cause disruption to the airflow in their vicinity whereby the particles of the aerosolized powdered medicament are deagglomerated due to the primary deagglomeration structure and further deagglomerated due to the secondary deagglomeration structure. The insert (100) defines a tortuous passage which has a trajectory with a centre line, whereby the centre line comprises two or more major bends, involving a first point of inflection between a pair of adjacent bends of the two or more major bends.

## Description

### TECHNICAL FIELD

The present disclosure generally relates to a dry powder inhalation device, and to an insert for a mouthpiece of a dry powder inhalation device.

### BACKGROUND

Dry powder inhalers (DPIs) are devices that are used by patients suffering from respiratory diseases in order to deliver medicines in the form of dry powders to lungs of the patient. In order to provide an effective dose of dry powder medicament, the powder must be deagglomerated during inhalation by the patient. Deagglomeration breaks up the powder into fine particles, able to travel deep into the lungs to deliver the dry powdered medicament to where it is most efficacious. Particles smaller than 5 microns in aerodynamic diameter are best able to penetrate the deeper regions of the human lung.

The dry powder medicament, as used in the dry powder inhalers, are however difficult to deagglomerate into the desired sub-5-micron particle size range. This is a result of the inherently high degree of cohesion between such small primary particles. Adhesion to an internal surface of the dry powder inhalers, sometimes known as "drug hold-up", is also an issue that affects drug delivery from the dry powder inhalation device. Drug hold-up is a problem, because the deposited drug that sticks to the dry powder inhalation device's internal surfaces is wasted. Over time it might also degrade and/or be released as large non-respirable lumps into subsequent inhalation airstreams which is clearly undesirable.

Typically, a fine particle fraction (FPF) may be increased by increasing resistance of the dry powder inhalers. Herein, a mass flow rate of air through the dry powder inhaler for a given patient-created pressure drop at the mouthpiece may be reduced. Unfortunately, increased resistance may be perceived as unpleasant by the patient. Furthermore, for ill patients who need efficient access to inhaled medication, increased inhaler resistance may be difficult to overcome, leading to reduced dose delivery and also to potential panic or respiratory distress. So, although increased resistance can increase FPF, it tends to lead to other problems. In fact, there is typically a trade-off in the dry powder inhaler's design for DPIs between three factors: the FPF produced, the resistance, and the drug hold-up.

Conventional refillable single-dose capsule DPI devices use the non-rotary type of deagglomerator, usually in the form of a screen or mesh that serves the dual purpose of retaining the powder-filled gelatine capsule and also of providing a measure of airflow shear induced deagglomeration. Such meshes have been used, for example, in commercially available capsule DPIs and can also be found in other proposed capsule inhaler designs. Such meshes can be metal or plastic. While simple and relatively cheap to manufacture, such inhalers are not particularly efficient, however. This is due in part to their fairly unsophisticated mesh deagglomerators.

In order to deagglomerate the primary particles of medicament, from one another and/or from any inert "carrier" materials such as coarser lactose particles, energy must be supplied to the dry powder inhalation inhalers. For a passive dry powder inhaler, that is, the dry powder inhaler which uses only the energy supplied by the patient's breath as they inhale, the energy is limited. Typically, deagglomerators in passive DPIs, that is, those DPIs that use energy from the patient's inhalation as the sole deagglomeration energy source, fall into one of two types. The first type creates an inhalation airstream that rotates in a helical manner as it travels towards a patient. Swirling of airflow in such DPIs, creates significant turbulence and shear within the inhalation airstream, leading to a measure of particle deagglomeration. In addition, use of contact surfaces, such as, those found in a helical mouthpiece deagglomeration system, leads to particle agglomerate collision and bouncing of particles at those surfaces. These collisions further deagglomerate particle agglomerates. Typically, such wall collisions are in fact more effective at breaking apart primary particles than is free space air shear. Unfortunately, however, such collisions can be a particular source of surface adhesion and hence increased unwanted drug hold-up in the inhaler. The second type of known DPI deagglomerator uses a different type of airflow passage geometry. In such deagglomerators, non-rotary motion is preferred. For example, one such system uses a baffle to deflect the inhaled powder-bearing airflow sufficiently to create substantial shear and collision deagglomeration within it. Such DPIs have been found to provide effective deagglomeration, but it still offers room for improvement in terms of the balance between FPF, device resistance and drug hold-up.

The state of the art shows a considerable number of dry powder inhalers, such as those addressed in the documents mentioned below.

CN110812635A discloses an inhaled dry powder inhalation dosing device having a first medicine carrying cavity and/or a second medicine carrying cavity for carrying a medicine bag are/is formed in a medicine carrying device. An air inlet is formed in the bottom of the first medicine carrying cavity and/or the second medicine carrying cavity, and the medicine carrying device comprises at least one first hole located in the left side face and/or at least one second hole located in the right side face, and a first boss is arranged on the left side face and/or a second boss is arranged on the right side face. One end of a needle is connected with a button, and the other end of the needle is slidably connected to the first hole and/or the second hole to puncture the medicine bag when overcoming the elastic force of an elastic element. A spiral device comprising a cylinder body and a blade structure is arranged in the cylinder body and used for generating vortexes. The blade structure is twisted by a preset angle in the vertical direction to form a spiral flow channel, and air from the air inlet carries the medicine powder from the medicine bag to sequentially pass through a grid flow channel, the spiral flow channel and a flat flow channel to enter the mouth.

WO2013/016787A1 discloses a powder inhaler, consisting of a base housing, snap-in capsule receptacle and a moveable mouthpiece, this latter internally including a device to open the capsule containing the powdered medication housed in a breakdown chamber, with air intake points and an outlet for air mixed with powder, with the outlet being integrated with the conduit of the mouthpiece. The above-mentioned passage for the air/powder mixture is located between the conduit and the chamber and is restricted and is sufficiently long to cause two effects in the capsule while it spins in the chamber during inhalation: a) valve-obstruction of the passage by the capsule, more precisely by its cylindrical part, briefly and intermittently, with such obstruction of the passage being repeated at every half turn (180°) of the capsule or whenever it is aligned with the passage; and b) impact of the capsule through suction against the roof of the breakdown chamber whenever it is aligned with the passage and its obstruction is complete, with this impact being sufficient to neutralize the compaction of the powder at the ends of the capsule during its rotation.

JP2011212269A provides a powder inhalation device capable of attaining smooth inhalation of a powder formulation taken out from a capsule for a long period. The powder inhalation device has a housing chamber for housing a capsule that is filled with the powder formulation, a body section having an outside air flowing channel for flowing outside air from an outside air taking in port to an outside air discharging port being an inhalation port of the powder formulation via the housing chamber, and a boring tool for boring a hole through the capsule C arranged in the housing chamber. The outside air flowing channel has a flowing out side channel which is arranged in a downstream side of the housing chamber, of which one end is connected to the outside air discharging port, and of which other end terminates by the side wall face in the body section, and there are boring tool passing channels for passing the boring tools through the housing chamber, connected to a peripheral wall face forming the flowing out side channel. There is a concave curbed face at least partially extending is arranged between the side wall face of the flowing outside channel and the peripheral wall face of the flowing out side channel.

US5469843A relates to a dry powder inhaler having one or more deagglomeration channels through which an airstream containing entrained medicament particles passes. Each channel has a substantially constant cross-sectional area and a bend having a radius of curvature of no greater than 10 mm.

WO2015/034709A1 discloses a dry-powder inhaler comprising a housing comprising at least one air inlet to a first air passage and a second air passage extending from the first air passage and terminating in a mouthpiece; the housing thereby providing a continuous air passage through the at least one air inlet, the first and second air passages, and the mouth piece; a rigid, fixed, powdered-medicament reservoir comprising a base, the reservoir being associated with the first air passage, and together with the first air passage forming a powder chamber; wherein air passing through the powder chamber travels substantially parallel to an upper surface of the reservoir and powdered-medicament positioned on the base in the reservoir, the upper surface of the reservoir having a surface area, and the reservoir having a volume.

WO2007/118490A1 provides a dispensing device for dispensing powder as a spray. The dispensing device comprises a duct through which the powder is dispensable by gas pressure for de-agglomerating the powder. The duct is angled by at least about 90 degrees at a diversion portion and/or diverted into two opposite directions at a fork portion so that the powder is impacted on to a solid surface for powder de-agglomeration.

US2020/230334A1 discloses a single-dose powder inhaler which is particularly compact and optimized for nasal application.

Therefore, in light of the foregoing discussion, there exists a need to overcome the aforementioned drawbacks associated with conventional DPI deagglomerators.

### SUMMARY

It is an object of the present invention to provide a dry powder inhalation device, which provides one or more solutions that overcomes at least partially the problems encountered in the prior art related to an appropriate balance between the FPF produced, the resistance, and the drug hold-up, or which at least provides the public with a useful choice.

In one aspect, an embodiment of the present disclosure provides a dry powder inhalation device, comprising a means to aerosolize powdered medicament and introduce the aerosolized medicament to an airflow directed to a user outlet for inhalation by a user, the device comprising a primary deagglomeration structure in the form of a baffle through which the airflow is arranged to pass downstream of the means to aerosolize the powdered medicament, and a secondary deagglomeration structure which is a tortuous passage through which the airflow is arranged to pass downstream of the primary deagglomeration structure,
the deagglomeration structures being configured to cause disruption to the airflow in their vicinity, whereby the particles of the aerosolized powdered medicament are deagglomerated due to the primary deagglomeration structure and further deagglomerated due to the secondary deagglomeration structure. As per the attached claims.

Embodiments of the present disclosure substantially eliminate or at least partially address the aforementioned problems in the prior art, by means of the disclosed dry powder inhalation device.

Additional aspects, advantages, features and objects of the present disclosure will be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF DRAWINGS

Embodiments of the present disclosure will now be described, by way of example only, with reference to the following diagrams wherein:
FIG.1A is a side view drawing of the outside of an insert in accordance with an embodiment of the present disclosure.
FIGs. 1B and 1C are perspective views of the insides of two halves of the insert of FIG. 1A in accordance with an embodiment of the present disclosure.
FIG. 1D is another perspective view (a side view) of the inside of the first half of the insert of FIG. 1B.
FIG. 1E shows a perspective view drawing of the outside of the first half of the insert of FIG. 1B.
FIG. 2A shows a sectioned view of a dry powder inhalation device (DPI) of the prior art, cut by a vertical plane.
FIG.2B shows another sectioned view of the DPI of the prior art shown in
FIG, 2A, from a different angle.
FIG. 3 shows a sectioned view of a DPI in accordance with the invention.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

The present disclosure provides a dry powder inhalation device. In the present embodiments, the dry powder inhalation device is a dry powder inhaler (DPI), as typically known, that may be used by patients suffering from respiratory diseases, such as asthma, bronchitis and the likes, to deliver medicament in a form of dry powder to lungs of the patient. Hereinafter, the terms "dry powder inhalation device," "DPI" and "dry powder inhaler" have generally been interchangeably used without any limitations.

The dry powder inhalation device comprises a means to aerosolize powdered medicament and introduce the aerosolized medicament to an airflow directed to a user outlet for inhalation by a user. Herein, powdered medicament may be medicament in powdered form that needs to be delivered to the user's lungs. Typically, the powdered medicament may be provided by a capsule placed in the dry powder inhalation device. The powdered medicament may be aerosolized by converting it to fine particles that are carried over by airflow due to their low inertia. In order to aerosolize the powdered medicament, the dry powder inhalation device may comprise tangential air inlets for air from surrounding to be sucked inside the dry powder inhalation device. The user outlet may be an opening or a mouthpiece that may be positioned between lips of the user for inhalation.

The means to aerosolize powdered medicament may be based upon one of any number of other refillable capsule DPI designs (e.g. Handihaler^{™}) or other non-capsule DPIs (e.g. Diskhaler^{™}, Diskus^{™}, Ellipta^{™}) or in new DPI device designs.

Air bypasses may be used deliberately for controlled reductions in overall inhaler airflow resistance and/or to provide alternative airflow shaping opportunities.

In a particular type of DPI, the user may open the dry powder inhalation device and place the capsule containing medicament into a "stadium" shaped recess. By "stadium" shaped recess, it is meant that the recess has a shape generally complementary to a shape of a typical capsule. A button may be provided on either side of the dry powder inhalation device that may be pressed in order to cause spring-loaded pins to be driven into ends of the capsule piercing them to leave two holes for subsequent air ingress and powder egress. The buttons may be then released and the springs may retract the pins from the capsule ends, leaving the capsule free to move during subsequent inhalation. The patient may then place the user outlet on his/her lips and may inhale air through it such that the airflow is directed towards the user outlet.

It may be appreciated that, in dry powder inhalation devices, the egressed powdered medicament must be deagglomerated, before being used by the patient. Deagglomeration breaks up the egressed powdered medicament from the capsule into fine particles so that the fine particles may be able to travel deep into the lungs where it is most efficacious. Typically, particles smaller than 5 microns in diameter are best able to penetrate deeper regions of the human lungs. A mass percentage of powdered medicament that is carried by such 5-micron particles is often referred to as a fine particle fraction (FPF). Since, the powdered medicament carried in sub-5-micron particles tends to be efficacious, whereas, larger particles tends to contribute more to unwanted side-effects and less to a therapeutic dose, it is important that the FPF produced by the dry powder inhalation device is both sufficient and consistent between individual users and between individual inhalations.

The dry powder inhalation device of the present disclosure comprises a primary deagglomeration structure in the form of a baffle through which the airflow is arranged to pass downstream of the means to aerosolize the powdered medicament. The baffle serves to divert airflow bearing the introduced aerosolized medicament sufficiently to create substantial shear and collision which in turn may cause deagglomeration of the aerosolized medicament in the vicinity of the baffle..

Optionally, the baffle is a mesh. The mesh is structure which is predominantly open to allow flow through it, having a small thickness in the flow direction. It could be constructed of filaments, that may be arranged in ordered or disordered fashion. The mesh may be made from wire or extruded plastic. It may be made as one piece with a multiplicity of through holes. Herein, the mesh may be an interlocked structure. Typically, the mesh may be positioned downstream of the aerosolization means. In capsule-based DPIs, a mesh is often used to retain the capsule. In a particular type of DPI, the mesh is positioned at a first end of an essentially disc-shaped swirl chamber. Herein, the first end may be above the said "stadium" shaped recess. The mesh may create the downstream airflow that is very turbulent. As airflow is directed towards the outlet, the mesh may serve to adjust the resistance to the airflow. The aerosolized powdered medicament introduced in the airflow may face resistance due to the mesh and hence, may be deagglomerated due to induced airflow shear.

The dry powder inhalation device further comprises a secondary deagglomeration structure which is a tortuous passage through which the airflow is arranged to pass downstream of the primary deagglomeration structure. The primary deagglomeration structure and the secondary deagglomeration structure may form a series combination. Herein, the primary deagglomeration structure may be followed by the secondary deagglomeration structure. The primary deagglomeration structure and the secondary deagglomeration structure may be structurally different, but may help with the same function of deagglomeration of the powdered medicament. Herein, the tortuous passage may be a passage having a plurality of twists and turns along its path. In one or more examples, the internal surface of the tortuous passage may be polished. It may be appreciated that as airflow passes through the tortuous passage, the powdered medicament may collide with internal surfaces (walls) at the twists therein. Hence, the aerosolized medicament introduced in the airflow may be further deagglomerated due to collision between particles of the aerosolized medicament itself and due to collision with internal surfaces of the tortuous passage, in the dry powder inhalation device.

The deagglomeration structures are configured to cause disruption to the airflow in their vicinity, whereby the particles of the aerosolized powdered medicament are deagglomerated due to the primary deagglomeration structure and further deagglomerated due to the secondary deagglomeration structure. As discussed, the primary deagglomeration structure in the form of the baffle is arranged so that the airflow through it passes downstream of the means to aerosolize the powdered medicament. The secondary deagglomeration structure which is a tortuous passage is arranged so that the airflow through it passes downstream of the primary deagglomeration structure. That is, first, the primary deagglomeration structure is arranged at a first end of the essentially disc-shaped swirl chamber. Next, the secondary deagglomeration structure is arranged downstream (next to) the primary deagglomeration structure. Hence, the airflow with the introduced aerosolized powdered medicament may be first deagglomerated due to the primary deagglomeration structure. This would break down the particles of the aerosolized powdered medicament. The particles may still be large and may be further deagglomerated by the secondary deagglomeration structure.

In embodiment, the secondary deagglomeration structure is an S-bend deagglomeration structure. In another embodiment, helical deagglomeration structure may be used as the secondary deagglomeration structure. In an embodiment, internal surface of deagglomeration structures may be modified to control drug hold-up. These include textured finishes to modify boundary layer fluid dynamics, ultra-smooth surfaces and/or low surface energy finishes to reduce drug adhesion, etc.

Optionally, the tortuous passage has a trajectory whereby a centre line of the trajectory comprises two or more major bends, involving a first point of inflection between a pair of adjacent bends of the two or more major bends. Herein, the trajectory is a path which is followed by the airflow, which is typically a curved path. The centre line is a line that traverses a central path through the trajectory. It may be defined as the line that traverses the 'centre of gravity' of the minimum cross-sectional areas cut through the trajectory. Herein, the two or more major bends may be two or more curves along the tortuous passage such that when two adjacent major bends are joined together at the first point of inflection, they may form an 'S' shape. For example, in an embodiment, the two or more major bends includes a first major bend and a second major bend. The first major bend may be in the shape of 'C' and the second major bend may be in the shape of a mirror image of 'C'. The first major bend and the second major bend may be joined at the first point of inflection between them in order to form the 'S' shape, and thus the first major bend and the major bend may together form the S-bend deagglomeration structure.

As the airflow passes through the major bends, the aerosolized powdered medicament introduced in the airflow deviates from its path and collides with the internal surfaces of the major bends. The aerosolized powdered medicament may also collide amongst themselves as their path deviates after collision. Such collisions may facilitate deagglomeration of the aerosolized powdered medicament. It may be noted that contrary, to the mesh, the S-bend deagglomeration structure may be more suitably used for the airflow that is laminar. However, the in-series combination of the mesh and the S-bend deagglomeration structure is capable of providing enhanced fine particle fraction (FPF) without leading to excessive drug hold-up or resistance. In an embodiment, fins, such as, fins with aerofoil sections may be added between the primary and secondary deagglomeration structures, in order to enhance the laminar nature of the airflow as it passes from the primary deagglomeration structure into the secondary deagglomeration structure.

It should also be appreciated that the exact nature and geometry of a transition region into the S-bend deagglomeration structure may be varied by one skilled in the art. Desirably, the transition region should be a gradual or a gentle transition so that the turbulent airflow emerging from the mesh is given space to become more laminar before being channelled into the S-bend deagglomeration structure. The science of fluid dynamics dictates that the transition region should be given as much space as possible, and experiments by inventors have determined that even a few extra millimetres may make a big difference. Conversely, a desire to have the secondary deagglomeration structure short enough to fit within a length of the dry powder inhalation device limits the length of the transition region.

Optionally, the major bends lie within a vertical plane or are generally aligned with a vertical plane. For reference, a vertical plane is considered to be a plane orientated vertically and in alignment with the user (i.e., towards/away from the user), when the user is inhaling through the DPI in the normal way. A horizontal plane is considered to be a plane orientated horizontally relative to the user inhaling through the DPI in the normal way. The centre line may lie in a vertical planeThis provides that the airflow is passed through the major bends and thus result in proper deagglomeration of the aerosolized powder medicament therein.

Optionally, the dry powder inhalation device involves a plurality of first points of inflection. The plurality of first points of inflection may be involved when there are more than two major bends. For example, in an embodiment, the centre line of the tortuous passage comprises four major bends. Herein, two major bends have one first point of inflection between them, as discussed, above. The other two major bends may also include one first points of inflection between them. It may be appreciated that more the number of major bends, the greater is the collision of the aerosolized powdered medicament with the internal surface of the major bends and hence, more is the deagglomeration of the aerosolized powdered medicament introduced in the airflow.

Optionally, the centre line of the trajectory comprises two or more lateral bends directed transversely to a plane containing or generally aligned with the two or more major bends, the two or more lateral bends involving a second point of inflection between a pair of adjacent bends of the two or more lateral bends. Similar to major bends, the lateral bends may also be curves along the tortuous passage, such that, when two adjacent lateral bends are joined together at the first point of inflection, they may form an 'S' shape. It may be appreciated that the two or more major bends may be considered to be in a Y plane. As discussed, the more the number of bends, the better is the deagglomeration of the aerosolized powdered medicament introduced in the airflow. Since, the two or major bends are considered to lie in the Y plane, in order to achieve better deagglomeration of the aerosolized powdered medicament, the centreline may comprise the two or more lateral bends directed transversely to the Y plane containing or generally aligned with the two or more major bends. Herein, the two or more lateral bends may be along an X plane and/or a Z plane, which are transverse to the Y plane. The introduction of the two or more lateral bends may further enhance the deagglomeration of the aerosolized powdered medicament introduced in the airflow. Thus, the lateral bends may introduce a degree of "yaw" to a general "pitch" nature of the airflow through the secondary deagglomeration structure. In a preferred embodiment, the yaw may be provided by steering the S-bend deagglomeration structure sidewards by 2-3 mm to one side and then back again to the other. This may create extra shear on the introduced aerosolized powdered particles as it traverses the secondary deagglomeration structure, leading to a further increase in FPF by typically, 2-3%.

Optionally, the at least one of the major bends and at least one of the lateral bends traverse a same portion of the centre line of the trajectory. As discussed, if the at least one of the major bends is considered in the Y plane, the at least one of the lateral bends may be transverse to the at least one of the major bends along X plane and/or Z plane. Herein, the at least one of the lateral bends in the centre line may be directed transversely from the plane of at least one of the major bends and positioned in the same region of the centre line as the at least one of the major bends. That is, the at least one of the lateral bends may be positioned upon the at least one of the major bends and thus may appear as a bump in the tortuous passage, in the the dry powder inhalation device. Furthermore, remaining portions of one of the lateral bends may be anywhere along the centreline outside the one of the major bends and may be directed transversely to the plane containing or generally aligned with the two or more major bends.

Optionally, substantially all of at least one of the major bends and substantially all of at least one of the lateral bends traverse the same portion of centre line of the trajectory. The tortuous path may be directed in a plane that is at an angle between that of major bends and lateral bends, resulting from the summation of major bends and lateral bends subtended by the same portion of centre line of the trajectory.

Optionally, the tortuous passage is an elongate conduit. Herein, the elongate conduit may be defined as an elongated channel such as a pipe, a tube and the likes, used for transporting the airflow along with the introduced aerosolized powdered medicament to the user outlet. It may be appreciated that the tortuous passage may be defined by bending and/or twisting of such elongate conduit.

Optionally, the elongate conduit has a cross section wherein the semi-minor axis or half-height is orientated in a plane containing or generally aligned with the major bends and is smaller than the semi-major axis or half-width. The cross section may be defined as an area transverse to the centre line of the trajectory. The semi-minor axis or half-height may be a maximum half-height dimension across a width of the conduit, along which the major bends are defined. The semi-major axis or half-width may be maximum half-width dimension across or transverse to the semi-minor axis along the cross section of the elongate conduit. As discussed, the elongate conduit may be the elongated channel used for transporting the airflow downstream towards the user outlet. The height of the elongate conduit may be measured along the centre line of the trajectory. The cross section of the elongate conduit may be transverse to the centre line of the trajectory. The cross section of the elongate conduit may have the semi-minor axis or half-height and the semi-major axis or half-width. The semi-major axis or half-width may be transverse to and larger than the semi-minor axis or half-height. The semi-minor axis or half-height is orientated in the plane containing or generally aligned with the major bends. It may be noted that, preferably, maximum dimension of the semi-major axis or half-width may be 9.6 mm and maximum dimension of the semi-minor axis or half-height may range between 2.2 to 2.3 mm. These dimensions may have been found to be particularly suitable for the S-bend deagglomeration structure for providing a desirable balance between the FPF, the resistance of the dry powder inhalation device and the drug hold-up, when tested with typical medicament formulation capsules at a typical user inhalation pressure drops such as, 0.5 kPa to 4 kPa.

It should be noted that dimensions such as, the semi-minor axis or half-height and the semi-major axis or half-width may be varied. One skilled in the art will appreciate the importance of varying dimensions slightly, while staying within the scope of this invention, in order to best achieve the desired objectives. For example, changing the semi-minor axis or half-height and the semi-major axis or half-width may affect its resistance to airflow. This may allow tuning of the dry powder inhalation device to a desired level for a particular pressure-drop required by the user. Such tuning may be beneficial when it comes to creating the dry powder inhalation device that is best matched to a given capsule medicament formulation. The tuning may also configure the secondary deagglomeration structures to be best suited according to the resistance or pressure drop characteristics of rest of the dry powder inhalation device.

Optionally, the elongate conduit has a cross section that is stadium-shaped. As used herein, the stadium-shape is similar to an oblong shape. In particular, herein, corners may be rounded similar to a football stadium. As the cross section of the elongate conduit may have the said stadium shape, this helps with the airflow while allowing for formation of bends therein, so as to allow for proper deagglomeration of the aerosolized powder medicament in the present dry powder inhalation device.

Optionally, the elongate conduit has a first twist along a first part of its trajectory, such that the first diameter deviates from alignment with the major bends, and in which the elongate conduit has a second twist, in the opposite sense to the first twist, along a subsequent part of its trajectory. In order to further enhance deagglomeration efficiency of the dry powder inhalation device, the first twist and the second twist may be formed along the trajectory. The first twist may be obtained by twisting the first part of the trajectory of the elongate conduit in one direction. The second twist may be obtained by twisting the subsequent part of the trajectory of the elongate conduit in direction opposite to that of the first twist. For example, if the first twist is obtained by twisting the elongate conduit along the first part in clockwise direction, the second twist may be obtained by twisting the elongate conduit along the subsequent part in anticlockwise direction. The first twist and the second twist may provide resistance to the airflow and the introduced aerosolized powdered medicament. Herein, the particles of the introduced aerosolized powdered medicament may collide with encountered inner surfaces due to the first twist and the second twist and hence, may be further deagglomerated. It may be noted that the first twist and the second twist may add a degree of "roll" to the general "pitch" nature of the airflow movement through the secondary deagglomeration structure. In a preferred embodiment, the roll is provided by adding a measure of helical twist to the S-bend deagglomeration structure. In another preferred embodiment, the roll is provided by adding a measure of helical twist to the S-bend in one direction which is the first twist followed by the measure of helical twist in the opposite direction which is the second twist.

Optionally, at least one of the major bends and at least one of the twists traverse a same portion of centre line of the trajectory. That is, herein, the same portion of centre line of the trajectory comprising the major bends may be twisted in opposite directions to obtain the first twist and the second twist. That is, for example, in an embodiment, the first part in a portion along the centre line of the trajectory comprising the major bends may be twisted in clockwise direction in order to obtain the first twist and the subsequent part of the same portion along the centre line of the trajectory comprising the major bends may be twisted in anticlockwise direction in order to obtain the second twist. Therefore, both the first twist and the second twist may be comprised in the same portion of the centre line of the trajectory.

Optionally, substantially all of at least one of the major bends and substantially all of at least one of the twists traverse the same portion of centre line of the trajectory. That is, herein, one of the major bends and one of the twists may traverse a portion of centre line of the trajectory, and the other major bends and the other twist may partially traverse the same portion of centre line of the trajectory. For example, in an embodiment, the first twist and first major bend may be comprised in the same portion of centre line of the trajectory, and the second twist and the second major bend may be partially comprised in the same portion of centre line of the trajectory.

In an embodiment, the centreline comprises two or more major bends, two or more lateral bonds, the first twist and the second twist. The inclusion of the two or more lateral bonds, the first twist and the second twist may add both the degree of "yaw" and the degree of "roll" to the general "pitch" nature of the airflow movement through the secondary deagglomeration structure.

In embodiments of the present disclosure, the dry powder inhalation device may be manufactured by moulding technique, such as injection moulding technique in which the dry powder inhalation device may be defined as two halves which are independently and individually injection moulded from a suitable plastic material such as acrylonitrile butadiene styrene (ABS) and then the two moulded halves are joined together (e.g. by the ultrasonic welding) to form the dry powder inhalation device. The two halves may be push-inserted into the bore of the mouthpiece to form the complete unit for the dry powder inhalation device. Alternatively, the insert's halves may be moulded from polypropylene (PP), and the like without any limitations. It may be noted that in order to mould halves of twisting conduit, cross section of radiused rectangular cross section of profile, may be moulded as a matching pair of profile halves that is split along "split surfaces". lines. Twisting profile may result in a different orientation of the cross section further along the profile. Further along the profile, outermost parts of the profile may have moved to a different part of the cross section, so the split surface changes. The stepped mating surfaces of the two moulded halves may form a labyrinthine seal to prevent air leaks when joined together. This is important, as inadvertent air leaks may reduce the airflow introduced with aerosolized powdered medicament that passes into a user's respiratory tract when inhaled by the user. Similarly, it is important that the insert is a tight fit within the bore of the DPI mouthpiece, both to avoid air leaks (and hence wasted inspiratory effort) and to ensure that the insert cannot become dislodged from the DPI mouthpiece and hence, be potentially swallowed or inhaled.

The present DPI may comprise the mouthpiece and the essentially disc-shaped swirl chamber. In a particular type of DPI of the disclosure, the user may swing the mouthpiece at the top and upper half aside of the essentially disc-shaped swirl chamber. Next, the user may place the medicament formulation contained in the capsule coated with captive gelatine into the "stadium" shaped recess below the essentially disc-shaped swirl chamber. Buttons may be provided on either side of the DPI that may be pressed causing spring-loaded pins to be driven into the ends of the captive gelatine capsule, piercing them to leave two holes for subsequent air ingress and powder egress. The user may then release the buttons, and the springs may retract the pins from the capsule's ends, leaving the capsule free to move during subsequent inhalation. The user may then place the mouthpiece between their lips and may inhale. The act of inhalation may cause reduction in air pressure inside the mouthpiece. Hence, the DPI may draw air in through the two tangential air inlets leading tangentially into the essentially disc-shaped swirl chamber that sits above the "stadium" shaped recess. The pressure reduction also draws the capsule into the essentially disc-shaped swirl chamber. Herein, the capsule starts to rotate under influence of the swirling airflow set up by the two tangential air inlets. Because the capsule is somewhat shorter than the diameter of the essentially disc-shaped swirl chamber, it may not simply spin around is centre, but instead it may follow a more chaotic uncontrolled motion, sometimes colliding with side walls of the essentially disc-shaped swirl chamber and sometimes flipping round and reversing. This chaotic motion ensures that the powdered medicament inside the capsule gets repeatedly disturbed by myriad different accelerations, decelerations and changes of direction. This causes the powdered medicament to be shaken loose into the airflow through the capsule between its pierced ends and then out of the capsule into the essentially disc-shaped swirl chamber and then through the baffle into the mouthpiece.

The present disclosure also provides an insert, for use in a mouthpiece of a dry powder inhaler (DPI). Herein, the insert defines a tortuous passage for an airflow directed to a user outlet for inhalation by a user, in which the tortuous passage has a trajectory with a centre line, whereby the centre line comprises two or more major bends, involving a first point of inflection between a pair of adjacent bends of the two or more major bends. The insert may be a device which when inserted into the mouthpiece of the dry powder inhaler defines the tortuous passage for the airflow. Herein, the insert may be the secondary deagglomeration structure for the DPI that may be added to an existing DPI to improve its FPF performance without excessive resistance or drug hold-up and without the need to modify the DPI. The tortuous passage is configured to help in deagglomeration of the airflow inside the dry powder inhaler (DPI) and direct the airflow to the user outlet for inhalation by the user. The user outlet may be positioned such that, when a user inhales on a DPI incorporating the insert, the user outlet is aligned with the user's mouth in order to inhale air through it. The tortuous passage has the trajectory with the centre line. Herein, the trajectory is the path followed by the airflow and the centre line is a line joining two ends of the trajectory. This centre line may be defined as the line that traverses the 'centre of gravity' of the minimum cross-sectional areas cut through the trajectory. The centre line comprises two or more major bends, involving the first point of inflection between the pair of adjacent bends of the two or more major bends. Herein, the two or major bends may be two or more curves. In an embodiment, the two or major bends may be in opposite directions. For example, in an embodiment, the two or more major bends may include the first major bend and the second major bend. The first major bend may be in the shape of 'C' and the second major bend may be in the shape of the mirror image of 'C'. The first major bend and the major bend may be joined at the first point of inflection between them in order to form the 'S' shape. In an embodiment, the insert may be in the form of the S-bend deagglomeration structure which pushes into the mouthpiece of the DPI and which is then held in place within said mouthpiece by means of an interference fit.

Optionally, the insert is fully insertable into the mouthpiece of a dry powder inhaler. Alternatively, the insert may be partially inserted into the mouthpiece of the dry powder inhaler, such that some portion of the insert is inserted into the mouthpiece and the other portion is outside the mouthpiece. The inserted portion is preferably seated in the DPI in a position to capture all of a dispensed and aerosolized powdered medicament dose.

Optionally, the centre line has three major bends, involving two first points of inflection respectively in airflow sequence between the first and second major bends and between the second and third major bends. The three major bends may be curves along the centreline. In an embodiment, the major bends may be similar and may be in shape of 'C'. The first and second major bends may be adjacent to each other and hence, may include one of the two first points of inflection between them. The second and third major bends may be adjacent to each other and hence, may include another first point of inflection between them. In another embodiment, the three major bends may be dissimilar curves. For example, the first major bend may be in the shape of 'C', the second major bend may be in the shape of the mirror image of 'C' and the third major bend may be again in the shape of 'C'. The first major bend and the second major bend may include one first point of inflection between them. The first major bend and the second major bend together may appear in the shape of 'S'. The second major bend and the third major bend may include another first point of inflection between them. The second major bend and the third major bend together may also appear in the shape of 'S'.

Optionally, the centre line of the trajectory comprises two or more lateral bends directed transversely to the two or more major bends, the two or more lateral bends involving a second point of inflection between a pair of adjacent bends of the two or more lateral bends. As discussed, the lateral bends may also be curves along the tortuous passage, such that, when two adjacent lateral bends are joined together at the second point of inflection, they may form the 'S' shape. The two or more lateral bends may be transverse to the two or more major bends. That is, if the two or more major bends are considered to be in the Y plane, the lateral bend may be along X plane and/or Z plane, which are transverse to the Y plane. The lateral bends may introduce the degree of "yaw" to the general "pitch" nature of the airflow through the insert. This may be achieved by steering the centreline, as it curves upwards and downwards, also from side to side, for example, by a total of 3 mm in one direction and then 3 mm back in other direction.

Optionally, the tortuous passage is an elongate conduit which has a first twist along a first part of its trajectory, such that the first diameter deviates from alignment with the major bends, and in which the elongate conduit has a second twist, in the opposite sense to the first twist, along a subsequent part of its trajectory. Herein, the elongate conduit may be the channel such as pipe, tube and the likes configured to transport the airflow towards the user outlet. Herein, the first twist may be obtained by twisting the first part of the trajectory of the elongate conduit in one direction. The second twist may be obtained by twisting the subsequent part of the trajectory of the elongate conduit in direction opposite to that of the first twist. For example, if the first twist is obtained by twisting the elongate conduit along the first part in clockwise direction, the second twist may be obtained by twisting the elongate conduit along the second part in anticlockwise direction. The first twist and the second twist may provide resistance to the airflow and the introduced aerosolized powdered medicament. Hence, the particle may collide with the first twist and the second twist and may be further deagglomerated. It may be noted that the first twist and the second twist may add the degree of "roll" to the general "pitch" nature of the airflow.

It may be noted that the insert may be formed by injection-moulding two halves. The two halves may be joined together by an ultrasonic welding of a suitable plastic material such as acrylonitrile butadiene styrene (ABS)), to form the insert which can be push-inserted into a bore of the mouthpiece of the DPI. Alternatively, the two halves may be moulded from polypropylene (PP). Two tangential air inlets may be provided in the insert. Air from environment may enter via a circular cross-sectional passageway which may funnel it downstream into passageway of the S-bend in the insert. This portion of the airflow passageway may have a constant or almost constant cross-sectional area, with its cross-sectional shape being similar to the oblong shape.

It should also be noted that the insert's two halves may be split in ways other than left-right split as discussed, above. For example, a top/bottom split, that is, with a curving split line surface running along the S-bend of the insert half away up its height is possible, albeit with constraints related to component moulding wall thicknesses. Such a split orientation has a potential benefit of removing a join line between the two parts from being along the centre line of the tortuous passage that deflect the airflow. If the join line is poorly formed, it might attract additional powder deposition. In such case, alternative approaches of splitting in ways other than left-right split as discussed, above may prove beneficial in reducing drug hold-up.

It may be further noted that a rotational alignment of the S-bend deagglomeration structure to the rest of the DPI may be controlled during factory insertion of the insert which is the S-bend deagglomeration structure. Although not critical to DPI function or performance, in general it may be preferable that maximum dimension of the cross-section of the conduit of the S-bend deagglomeration structure is aligned horizontally, in order that the emerging flow of airflow introduced with the aerosolized powdered medicament is best aligned with the shape of a user's mouth and buccal cavity.

The dry powder inhalation device and the insert of the present disclosure are advantageous in efficient deagglomeration of particles so that the drug hold-up is reduced and particles are deagglomerated small enough to be respirable into the lungs even at lower resistance. In the present dry powder inhalation device, the volume of powdered medicament delivered from 20mg-filled capsules at an inhalation flow rate of 40 L/min for 6 seconds to the user in percentage for 5 microns particles has a particularly good performance improvement for the lower resistance configuration, with an uplift in fine particle fraction (FPF) from 35.5% to 51.6% under the same testing conditions with existing devices. Moreover, the secondary deagglomeration structure of the dry powder inhalation device provides a reliable way to substantially improve pharmaceutical drug delivery performance of the capsules. Furthermore, the insert of the present disclosure may be inserted in conventional DPIs in order to improve their performance by requiring only simple linear insertion of a pre-assembly of two moulded halves into a full length of the mouthpiece of the conventional DPIs.

### DETAILED DESCRIPTION OF DRAWINGS

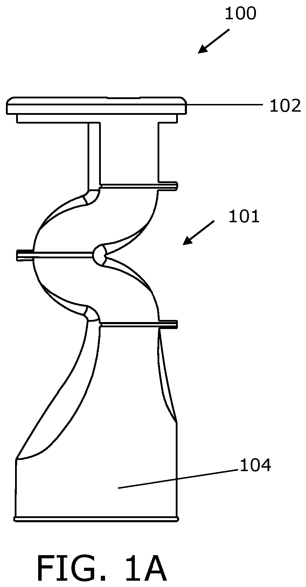

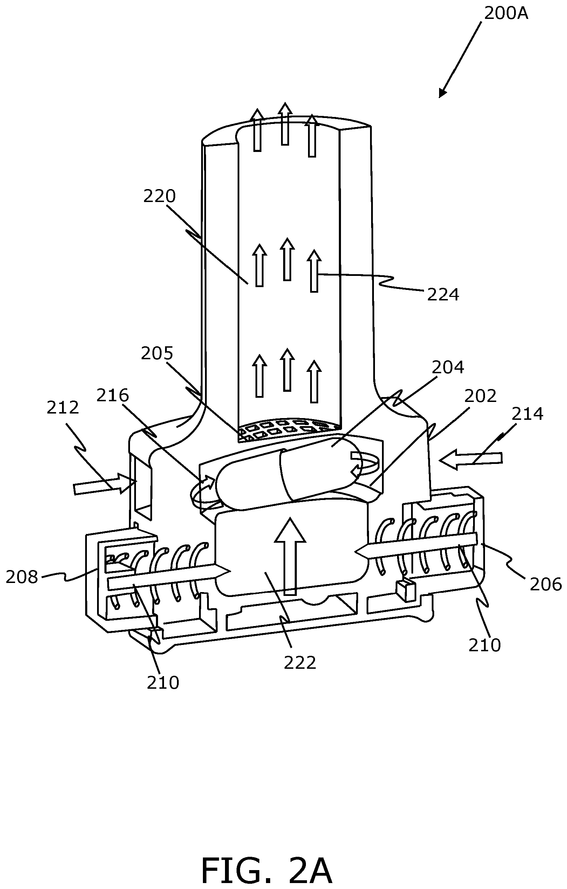

FIG.1A is a side view drawing of the outside of an insert in accordance with an embodiment of the present disclosure. The insert 100 comprises a secondary deagglomeration structure 101. The insert has a distal end 102 for seating the insert 100 inside the mouthpiece opening of a dry powder inhalation device, such as one shown in Fig. 2A. 2B. The dry powder inhalation device typically has a primary deagglomeration structure (e.g. 205 in FIG. 2A).

The insert 100 comprises a proximal end 104, which is inserted deeply into the mouthpiece of the dry powder inhalation device to seat in a position to capture all of a dispensed and aerosolized powdered medicament dose.

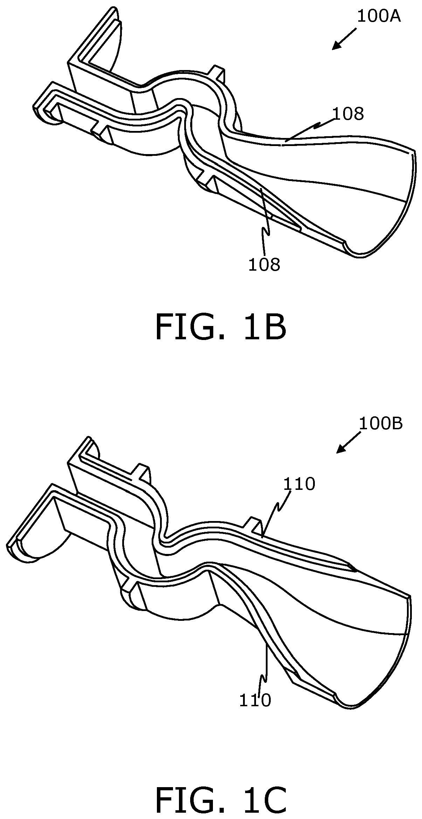

FIGs. 1B and 1C are perspective views of the insides of two halves of the insert of FIG. 1A in accordance with an embodiment of the present disclosure. FIG. 1B shows a first half 100A and FIG.1C shows a second half 100B, of the insert 100. Herein, the first half 100A and the second half 100B are joined together to form the insert 100. The first half 100A and the second half 100B are formed by moulding and defined as splitting the secondary deagglomeration structure along a left-right direction. The first half 100A has a first stepped mating surface 108 and the second half 110B a second stepped mating surface 110. The first stepped mating surface 108 and the second stepped mating surfaces 110 form a labyrinthine seal to prevent air leaks when the first half 100A and the second half 100B are joined together. The secondary deagglomeration structure 101 is formed when the first half 100A and the second half 100B are joined together.

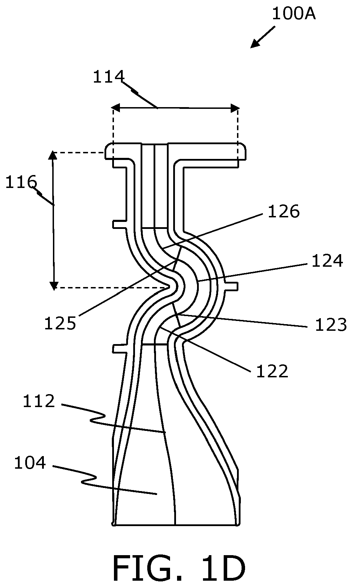

FIG. 1D is another perspective view (a side view) of the inside of the first half of the insert of FIG. 1B. A centre line 112 is shown and allows the bend arrangement of the secondary deagglomeration structure to be defined. The centre line describes a first bend 122 with a deviation of approximately 60°, merging into a second bend 124 with a deviation in the opposite sense of approximately 120°, via a point of inflection 123. The centre line continues to describe the second bend 124 merging into a third bend 126, with a deviation in the opposite sense to that of the second bend, of approximately 60°, via a point of inflection 125. Since this secondary deagglomeration structure is operating essentially in one plane, both of the points of inflection are "first" points of inflection, even though they occur in the above-described sequence. Exemplary dimensions for the insert include a width 114, corresponding to the internal diameter of the mouthpiece of the dry powder inhalation device to be coupled, in the range 10.86±0.05 mm. The longitudinal distance from the seating at the distal end 102 to the centre of the second bend 124 may be in the range 11.60±0.05 mm.

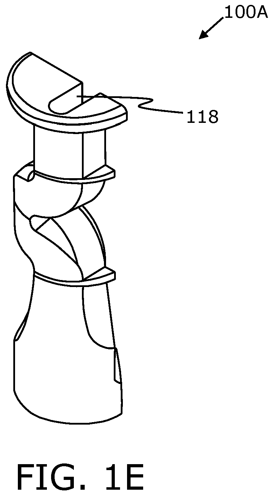

FIG. 1E shows a perspective view drawing of the outside of the first half of the insert 100A. It shows a corresponding half of a user outlet 118. The user outlet provides the exit for deagglomerated medicament dose, when a user inhales on the mouthpiece of the dry powder inhalation device.

FIG. 2A shows a sectioned view 200A of a dry powder inhalation device (DPI) of the prior art, cut by a vertical plane. The DPI has an essentially disc-shaped swirl chamber 202, two buttons 206 and 208, spring-loaded pins 210, air inlets 212 and 214 that lead tangentially to the disc-shaped swirl chamber, a mesh baffle 205, a mouthpiece 220, and a "stadium" shaped recess 222 below the circular swirl chamber.

To use the DPI, the patient swings the mouthpiece 220 and swirl chamber upper half aside, and then places a medicament formulation-containing capsule 204 containing powdered medicament into the recess 222. The ends of the capsule are punctured by the user pressing buttons 206, 208 that drive the pins 210 into ends of the capsule 204. The pins 210 are spring-loaded to retract when the use releases the buttons 206, 208. When a user inhales on the mouthpiece 220, air is drawn through inlets 212, 214. This creates a swirling motion of low-pressure air that draws the capsule from recess 222 into swirl chamber 202. The capsule 204 is a loose fit inside the in the swirl chamber 202; so, it undergoes a chaotic uncontrolled spinning motion (indicated by arrows 216), expelling the powder as it goes. The capsule 204 is retained by the mesh baffle 205, and the powder passes through the mesh baffle and out through the mouthpiece 220. The motion of air carrying the powder is indicated by arrows 224.

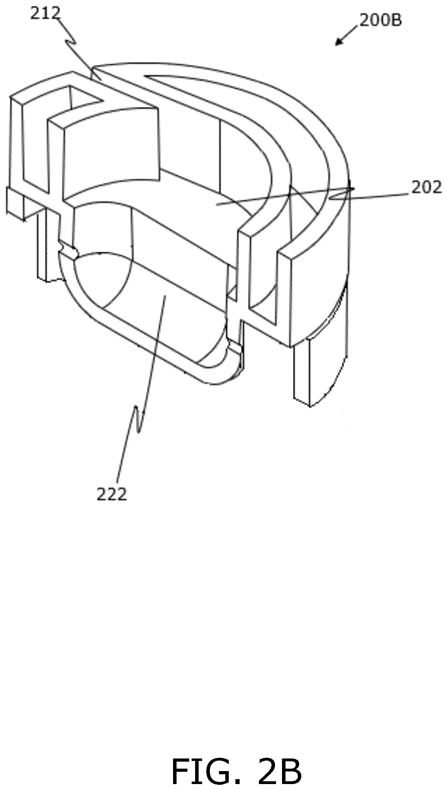

FIG.2B shows another sectioned view 200B of the DPI of the prior art shown in FIG, 2A, from a different angle. In the drawing, an additional cut across a horizontal plane has been made, and the spring-loaded pins and buttons removed. This better shows the shapes of the chamber 202, the recess 222 and one tangential inlet 212.

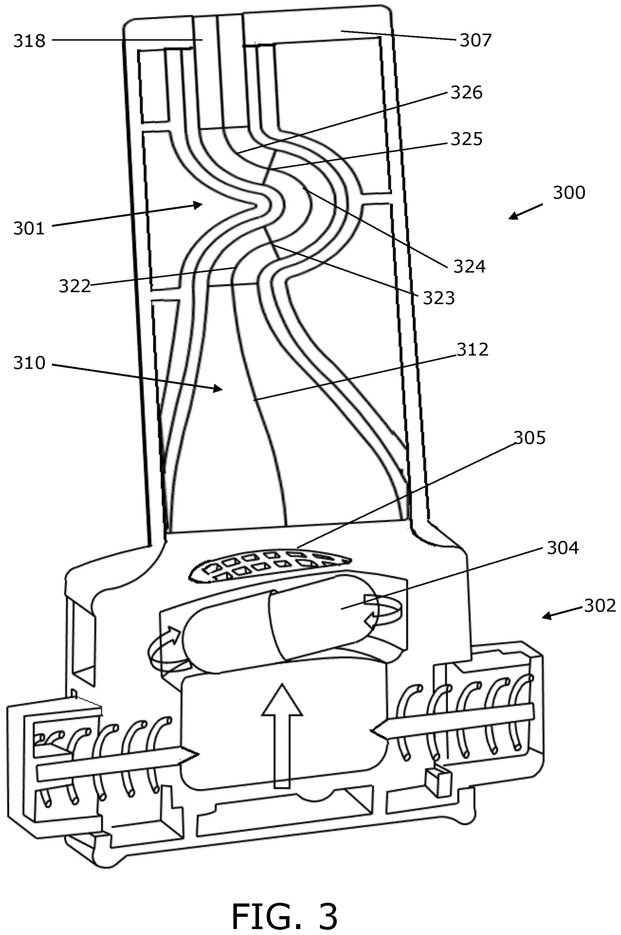

FIG. 3 shows a sectioned view 300 of a DPI in accordance with the invention. The DPI has a means 302 to aerosolize powdered medicament from capsule 304, a first deagglomeration structure in the form of a mesh 305, a tortuous passage 310, and a mouthpiece 307 with a user outlet 318. Thus, an insert similar to that shown in FIG. 1 has been incorporated as part of the DPI. The tortuous path trajectory is characterized by a centre line 312. The tortuous path provides the secondary deagglomeration structure 301. As in FIG. 1D, the centre line of the tortuous path has, in sequence, a first bend 322, a first point of inflection 323, a second bend 324, a second point of inflection 325 and a third bend 326. When a user inhales on the mouthpiece 307, air is drawn into the means 302 to aerosolize powdered medicament. The powdered medicament is released in aerosolized form. As it is drawn through the first deagglomeration structure, the particle size of the powder is reduced. The powder is then carried through the tortuous path 310, whereupon the particle size is reduced yet further, before exiting the user outlet 318.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Expressions such as "may" and "can" are used to indicate optional features, unless indicated otherwise in the foregoing. Reference to the singular is also to be construed to relate to the plural.

## Claims

1. An insert (100) for use in a mouthpiece (307) of a dry powder inhaler (300), the insert defining a tortuous passage (310) for an airflow directed to a user outlet (318) for inhalation by a user, in which the tortuous passage (310) has a trajectory with a centre line, whereby the centre line (312) comprises two or more major bends (322, 324, 326), involving a first point of inflection (123, 323) between a pair of adjacent bends of the two or more major bends.

2. An insert (100) according to claim 1 in which the centre line (312) of the trajectory comprises two or more lateral bends directed transversely to the two or more major bends (322, 324, 326), the two or more lateral bends involving a second point of inflection (125, 325) between a pair of adjacent bends of the two or more lateral bends.

3. An insert (100) according to claim 1 or claim 2, in which the tortuous passage (310) is an elongate conduit which has a first twist along a first part of its trajectory, such that the first diameter deviates from alignment with the major bends (322, 324, 326), and in which the elongate conduit has a second twist, in the opposite sense to the first twist, along a subsequent part of its trajectory.

4. An insert (100) according to any one of claims 1 to 3, which is fully insertable into the mouthpiece (307) of a dry powder inhaler (300).

5. An insert (100) according to claim 1, whereby the centre line (312) has three major bends (322, 324, 326), involving two first points of inflection (123, 323) respectively in airflow sequence between the first and second major bends and between the second and third major bends.
